# FASCICULE DE BREVET EUROPEEN

(11) **EP 0 514 449 B1**
(45) Date de publication et mention de la délivrance du brevet: **14.05.1997**
(21) Numéro de dépôt: 91904092.3
(22) Date de dépôt: 06.02.1991
(51) Int. Cl.: A61K 31/72, A61K 31/735

(54) **NOUVEL AGENT ANTITUMORAL DERIVE DU DEXTRANE**
DEXTRANVERBINDUNG ALS ANTITUMORMITTEL
NOVEL ANTI-TUMORAL AGENT DERIVED FROM DEXTRAN

(30) Priorité: 06.02.1990 FR 9001343
(43) Date de publication de la demande: 25.11.1992
(73) Titulaire: THERAPEUTIQUES SUBSTITUTIVES, F-93430 Villetaneuse (FR)
(72) Inventeur: JOZEFOWICZ, Jacqueline, F-60260 Lamorlaye (FR); HARMAND, Marie-Françoise, F-33000 Bordeaux (FR); SLAOUI, Faouzi, Meknes (MA)
(74) Mandataire: Ores, Irène
(86) Numéro de dépôt international: FR9100092
(87) Numéro de publication internationale: WO9112011

(56) Documents cités:
- FR-A- 2 555 589
- Molecular Immunology, vol. 25, no. 2, 23 Febr. 1988; M.P. Carreno et al., pp. 165-171
- WPI, File Supplier, AN=78-75503A, Derwent Publications Ltd, Londres, GB; & JP-A-53 105 584

## Description

La présente invention est relative à des agents inhibant la croissance des cellules tumorales, et à leur application pour l'obtention de médicaments antitumoraux.

Il existe plusieurs substances, d'origine et de composition variées, qui possèdent une activité antitumorale ; toutefois, la grande diversité des tumeurs existantes d'une part, et d'autre part les effets secondaires desdites substances, qui découlent de leur toxicité souvent importante vis-à-vis des cellules saines, et les variations qui peuvent exister d'une tumeur à l'autre, et d'un individu à l'autre dans la réponse à un traitement par une substance donnée, impliquent la nécessité de disposer de produits antitumoraux très variés, permettant des traitements adaptables à chaque cas. S'il existe actuellement plusieurs médicaments dotés d'effet antitumoraux (une quarantaine de substances sont au-jourd'hui utilisées à cet effet), trop nombreux sont encore les médicaments dont l'efficacité est insuffisante ou aléatoire. La mise au point de nouveaux agents antitumoraux présente donc un intérêt thérapeutique considérable.

D'autre part, il est connu, par exemple par le Brevet Français 2 461 724, et le Brevet français 2 555 589, que la substitution de polymères tels que les dextranes par des chaînes latérales portant des groupes carboxyliques et sulfonate, leur confère des propriétés anticoagulantes.

Les Inventeurs ont maintenant mis en évidence de nouvelles propriétés de certains dextranes substitués par des chaînes latérales portant des groupes carboxyliques et sulfonate ; ils ont en effet découvert que pour certaines valeurs de taux de substitution par lesdits groupes, lesdits dextranes substitués s'avéraient de bons inhibiteurs de la croissance des cellules tumorales, n'ayant en outre que peu ou pas d'effet sur la croissance des cellules saines.

La présente invention a pour objet l'utilisation d'un dérivé de dextrane constitué par une chaîne polysaccharidique substituée par des groupes carboxyméthyle et carboxyméthylbenzylamide sulfonate, lequel dérivé est désigné par la formule générale D_{X}CM_{Y}BS_{Z} dans laquelle
- X: représente le nombre moyen d'unités saccharidiques non substituées pour 1OO unités saccharidiques,
- Y: représente le nombre moyen de groupes carboxyméthyle pour 1OO unités saccharidiques,
- Z: représente le nombre moyen de groupes carboxyméthylbenzylamide sulfonate pour 1OO unités saccharidiques,
et X est inférieur ou égal à 50, Y est compris entre 10 et 90, et Z est compris entre 15 et 35, pour l'obtention d'un agent inhibiteur de la croissance des cellules tumorales.

Selon un mode de réalisation préféré de la présente invention, X est égal à 11, Y est égal à 60 et Z est égal à 29.

Selon un autre mode de réalisation préféré de la présente invention, X est égal à 47, Y est égal à 17 et Z est égal à 24.

L'invention sera mieux comprise à l'aide du complément de description qui va suivre, qui se réfère à des exemples de préparation et d'utilisation des agents antitumoraux conformes à l'invention.

Il doit être bien entendu, toutefois, que ces exemples sont donnés uniquement à titre d'illustration de l'objet de l'invention.

### EXEMPLES

### I - PREPARATION DES AGENTS INHIBITEURS DE LA CROISSANCE DES CELLULES TUMORALES CONFORMES A L'INVENTION

Des dextranes fonctionnalisés par des groupes carboxyméthylbenzylamide sulfonate (DCMBS) sont préparés selon le procédé décrit dans la publication de MAUZAC et al. [Biomaterials, 3, (1982)], c'est à dire en 3 étapes : carboxyméthylation du dextrane, couplage de la benzylamine sur les groupes carboxyméthyle, sulfonatation des noyaux aromatiques des groupes carboxyméthyl-benzylamide.

Des dextranes fonctionnalisés par des groupes carboxyméthyle (DCM), et carboxyméthylbenzylamide (DCMB), sont également préparés, afin de comparer leur activité antitumorale à celle des dérivés conformes à l'invention.

Des dextranes fonctionnalisés sont désignés par la formule générale D_{X}CM_{Y}B_{Z}'S_{Z} dans laquelle
- X: représente le nombre moyen d'unités saccharidiques non substituées, pour 100 unités saccharidiques
- Y: représente le nombre moyen de groupes carboxyméthyl pour 100 unités saccharidiques
- Z': représente le nombre moyen de groupes carboxyméthyl benzylamide pour 100 unités saccharidiques
- Z: représente le nombre moyen de groupes carboxyméthylbenzylamide sulfonate pour 100 saccharidiques.

Pour l'obtention des dextranes fonctionnalisés conformes à la présente invention, X doit être inférieur ou égal à 50, Y doit être compris entre 10 et 90, Z' doit être au plus égal à 5, et Z doit être compris entre 15 et 35.

Ces valeurs sont obtenues en faisant varier différents paramètres dans le procédé de préparation des dérivés de dextrane ; la façon de procéder au choix des conditions de chaque étape de la réaction est décrite ci-après :

### a) Carboxyméthylation

Dérivée de la réaction effectuée sur la cellulose pour obtenir la carboxyméthylcellulose, cette opération est réalisée en milieu basique par action à froid de l'acide monochloroacétique ClCH₂COOH sur le dextrane Dx(OH)₃. R, le rapport carboxyméthylant, est le nombre de moles d'acide monochloroacétique introduit dans le milieu réactionnel par motif de dextrane présent dans ce milieu. Ce rapport contrôle la réaction de carboxyméthylation, mais dans le cas du dextrane, il n'est pas possible d'utiliser des rapports carboxyméthylants élevés, au risque de dégrader la chaîne macromoléculaire du dextrane. Il est donc indispensable de procéder à des carboxyméthylations successives à des faibles valeurs de R, (au plus égales à 3,5) pour arriver aux pourcentages de substitution souhaités lorsqu'ils sont élevés. Le temps de réaction et la température du milieu réactionnel ont aussi un effet sur le rendement de la réaction, c'est-à-dire sur le nombre de motifs dextranes portant un groupe carboxyméthyle pour 100 motifs.

Pour une valeur de R = 3,5, à une température de 5O°C, et pour un temps de réaction de 40 mn, on obtient un rendement de réaction de l'ordre de 60%.

Pour obtenir un polymère ayant un pourcentage de substitution voisinant 100% en maintenant ces conditions de synthèse, il est indispensable de procéder à des carboxyméthylations successives. L'on peut ainsi atteindre des valeurs supérieures à 100% d'unités substituées sur deux de leurs fonctions hydroxyles. Toutefois, à partir de la troisième étape de synthèse, l'augmentation du pourcentage de substitution est faible puisqu'il est de 5 à 10% entre la troisième et la quatrième étape.

### b) Couplage de la benzylamine sur les groupes carboxyméthyle :

Le principe de cette réaction classique est fondé sur l'aptitude de la fonction carboxylique à former un anhydride mixte instable capable de réagir avec un réactif portant une fonction amine primaire (R-NH2). Deux procédés différents communément dénommés réactions d'activation ont été utilisés pour aboutir à la formation d'un anhydride mixte :
- action du chloroformate d'isobutyle (CIB)
- action du N-Ethoxycarbonyl-2-Ethoxy-1-2-Dihydroquinoléine (EEDQ).

### Couplage au CIB

Le couplage proprement dit consiste alors à faire réagir l'amine primaire, en l'occurrence la benzylamine, sur la fonction anhydride mixte instable.

Cette réaction est effectuée dans un mélange eau/diméthylformamide (DMF). Les proportions 50/50 en volume eau/DMF ont été retenues car c'est le mélange qui pose le moins de problèmes de solubilité des réactifs et qui permet un bon rendement. Les rapports molaires : CIB/-COOH, B/-COOH et NMM/-COOH retenus sont de 2.

L'activation doit être effectuée, à pH 3,5 pour empêcher le dextrane de précipiter, en un temps très rapide, et à faible température (1 minute à -15°C) puisque l'anhydride mixte, très instable, se décompose par élévation de la température. La réaction de couplage est effectuée pendant 30 minutes à -15°C, puis 1 heure à 20°C. A la fin de la réaction, l'eau est éliminée par évaporation du milieu réactionnel puis le produit est précipité et purifié. Le carboxyméthylbenzylamide-dextrane (CMDB), ainsi synthétisé subira un deuxième et/ou un troisième couplage pour améliorer le rendement de substitution.

### Couplage à l'EEDQ

Dans ce cas, l'étape de couplage de la benzylamine est effectuée dans un mélange eau/éthanol de manière à assurer la solubilité du mélange. Le rapport eau/éthanol retenu est de 1,2:1 en volume.

La solution de EEDQ doit être ajoutée à la solution aqueuse de carboxyméthyldextrane sous agitation continue ; le pH est maintenu entre 3,5 et 4,5 à l'aide d'une solution d'HCl 1M ; l'agitation est maintenue pendant environ 30mn à température ambiante ; il est toute-fois avantageux, si l'on souhaite obtenir un rendement de substitution élevé (jusqu'à 30%), d'opérer à une température de 30 à 40°C ce qui augmente l'activation du produit intermédiaire.

Un volume de benzylamine est alors ajouté au mélange, préalablement refroidi, si nécessaire, à une température d'environ 20°C ; le rapport molaire benzylamine/EEDQ est égal à 1. L'addition de soude est nécessaire au cours de la réaction pour maintenir le milieu basique et améliorer de ce fait le couplage en maintenant le pH apparent à une valeur voisine de 9. Cette opération est effectuée par contrôle continu du pH apparent avec une électrode de verre. La réaction est effectuée pendant une durée variant de 8 à 17 heures.

Les deux procédés de couplage donnent des pourcentages de substitution semblables avoisinant 8 à 12% en une seule étape. Dans le cas du couplage par l'EEDQ, ce rendement peut atteindre 25 à 30%, en une seule étape, lorsque l'on procède à l'activation du produit intermédiaire à une température de 30 à 40°C.

Comme pour la carboxyméthylation, lorsque les pourcentages de substitution de la benzylamine souhaités sont élevés, il n'est pas possible d'augmenter les concentrations des différents réactifs. Il est donc nécessaire de faire des couplages successifs pour améliorer le rendement de la réaction. Le CMDB précipité lavé et séché après le premier couplage va subir un second et/ou un troisième couplage exactement dans les mêmes conditions que le premier sans considération des substitutions dues au premier couplage.

### c) Sulfonatation des noyaux aromatiques

Elle se fait par action de l'acide monochlorosulfonique sur le CMDB en milieu organique anhydre (dichlorométhane par exemple) et en phase hétérogène, le CMDB n'étant pas soluble dans le dichlorométhane. Il est nécessaire d'opérer la réaction en excès d'acide monochlorosulfonique sans pour autant risquer, d'une part, une hydrolyse acide de la chaîne polysaccharidique et, d'autre part, une sulfonatation des fonctions hydroxyle portées par les unités glucosyles, qui donnerait des fonctions sulfate. Le rapport molaire R'=[HSO₃Cl]/[benzylamide] où la concentration molaire en benzylamide [benzylamide] est celle de la benzylamide, fixée au polymère qui subit la réaction de sulfonation, doit être égal à 3. La concentration de l'acide chlorosulfonique dans le milieu réactionnel ne doit pas dépasser 0,15 M.

Dans ces conditions, le pourcentage d'unités portant une fonction sulfonate dépend du pourcentage d'unités substituées par des groupes benzylamide.

En effet, plus le dextrane est riche en fonctions benzylamide, meilleur est le rendement de la sulfonatation. Ainsi, il est possible d'obtenir des dérivés dont le pourcentage d'unités portant la fonction sulfonate est de 35% quand le CMDB sur lequel la sulfonatation a été effectuée présente près de 35% d'unités saccharidiques portant une fonction benzylamide.

En effet, aux conditions de concentration précédemment décrites les fonctions sulfonate se fixent préférentiellement sur les noyaux aromatiques des fonctions benzylamide et non pas au niveau des fonctions hydroxyle présentes sur le cycle des dextranes.

Les sulfonatations successives, au-delà de la deuxième ou de la troisième selon les cas, n'améliorent pas le rendement de la substitution mais, au contraire, entraînent une dégradation du polymère.

### II - COMPARAISON DES EFFETS DES AGENTS ANTITUMORAUX CONFORMES A L'INVENTION, ET DE DIFFERENTS DERIVES DE POLYSACCHARIDES SUR LA CROISSANCE DE LIGNEES CELLULAIRES TUMORALES ("CHONDROCYTES" ISSUS DE CHONDROSARCOME HUMAIN).

Les effets des agents antitumoraux conformes à l'invention ont été étudiés en comparaison avec, d'une part, ceux de polysaccharides non sulfatés : le dextrane natif T 40, et des dextranes modifiés de type CMB, d'autre part, ceux d'un polysaccharide sulfaté : l'héparine, et enfin, ceux de dérivés du type DCMBS portant moins de 15% d'unités saccharidiques substituées par des groupes sulfonate.

### A) Protocole pour l'étude de la synthèse de l'ADN:

L'étude de la synthèse de l'ADN est réalisée à l'aide d'un composé radio-marqué : la thymidine tritiée. Pour cela, un protocole adapté aux chondrocytes normaux et tumoraux a été mis au point.

Les tests sont réalisés dans des boîtes de culture de 96 puits. Les cellules sont ensemencées en présence de 10% SVF (Sérum de Veau Foetal) à une concentration de 20 000 cellules/ml ; 200 µl de cette suspension cellulaire sont distribués dans les puits. Après une période de 7 à 10 jours, lorsque les cellules sont à confluence, elles sont progressivement privées de sérum (de façon à arriver à une concentration de sérum dans le milieu de 0,1%). Les substances à tester sont ajoutées dans les puits, à des concentrations variées ainsi que la thymidine tritiée (1 µCi/puits). L'incorporation se déroule pendant 48 heures. Pour chaque dose testée, 8 à 10 puits sont étudiés. A la fin de l'incorporation, la couche cellulaire est lavée avec une solution de tampon phosphate (PBS) puis fixée deux fois pendant 5 minutes en présence de méthanol 5%, lavée 4 fois avec 200 µl de PBS, puis précipitée en présence d'acide trichloracétique (TCA) 5% deux fois pendant 10 minutes à 4°C. Après un dernier lavage, la couche cellulaire est dissoute par 200 µl d'une solution de NaOH 0,3 M. Les 200 µl sont prélevés, transférés dans un tube à scintillation, additionnés de 5 ml de liquide scintillant. La radioactivité des tubes est déterminée dans un compteur à scintillation.

### B) Etude des effets des différents dérivés de polysaccharides sur la croissance des cellules.

Ces effets ont été étudiés parallèlement, à deux concentrations de sérum de veau foetal, respectivement de 0,5% et de 5%.

### 1) Dérivés ne portant pas de fonctions sulfate libres :

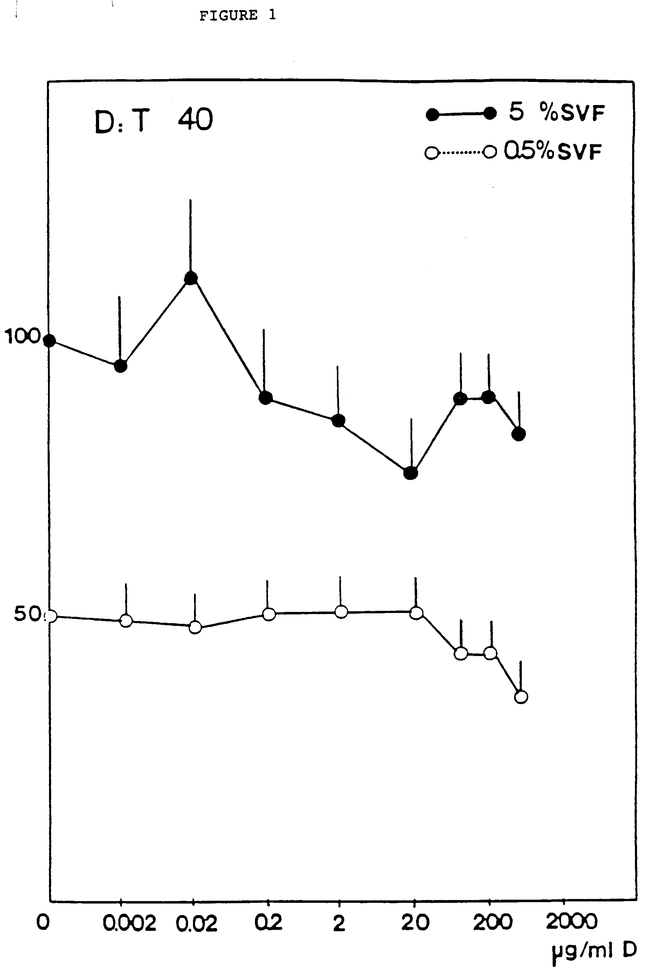

### EXEMPLE 1 (Exemple de comparaison) : effets du dextrane T 40 (Figure 1)

On observe une inhibition de 10% (P<O,05) de l'incorporation de thymidine tritiée aux fortes concentrations (> 20 µg/ml) en présence de faibles concentrations de SVF (0,5%) (○).

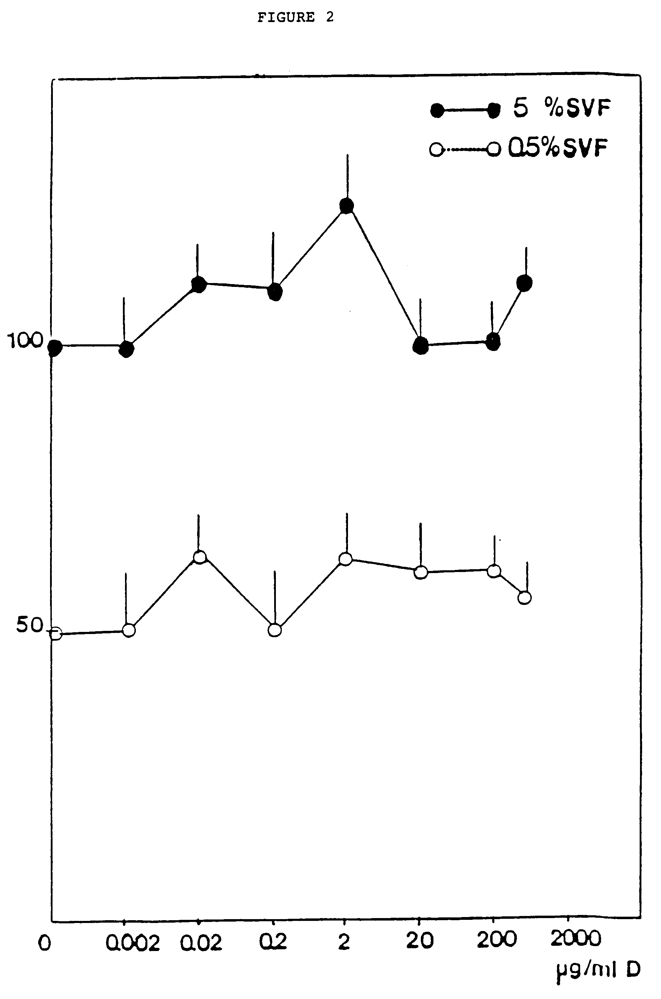

### EXEMPLE 2 (exemple de comparaison) : effets du dextrane substitué D₀CM₈₄B₂₁S₀ (●) (Figure 2)

On observe, en présence de 5% SVF, une stimulation de l'incorporation d'environ 10% (P<O,05) pour des concentrations de 20 et 200 ng/ml, et de 25% (P<O,01) pour 2 µg/ml de D_{O}CM₈₄B₂₁S_{O}. Pour les concentrations plus élevées, l'incorporation n'est pas modifiée.

En présence de faibles concentrations de sérum (0,5%) (○), on observe une stimulation d'environ 10% (P<O,05), pour les concentrations de D_{O}CM₈₄B₂₁S_{O} supérieures ou égales à 20 ng/ml.

### 2) Dérivés portant des fonctions sulfate et sulfamate libres :

### EXEMPLE 3 (exemple de comparaison) : Effets de l'héparine

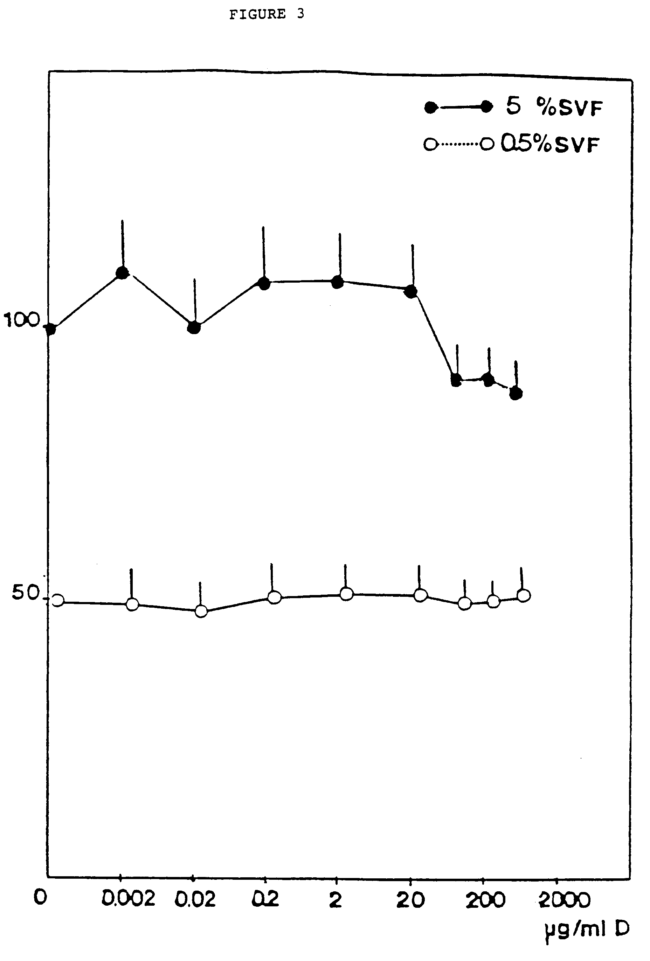

La Figure 3 représente l'effet de l'héparine H 108 (fournie par l'Institut CHOAY) au niveau des chondrocytes tumoraux (typeII). On observe peu de modifications de l'incorporation en présence d'héparine quelles que soient les concentrations testées. Seules les concentrations élevées (100 à 400 µg/ml) diminuent l'incorporation d'environ 10% (P<O,02) en présence de 5% (●) de SVF.

### 3) Dérivés de dextrane portant des fonctions sulfonate

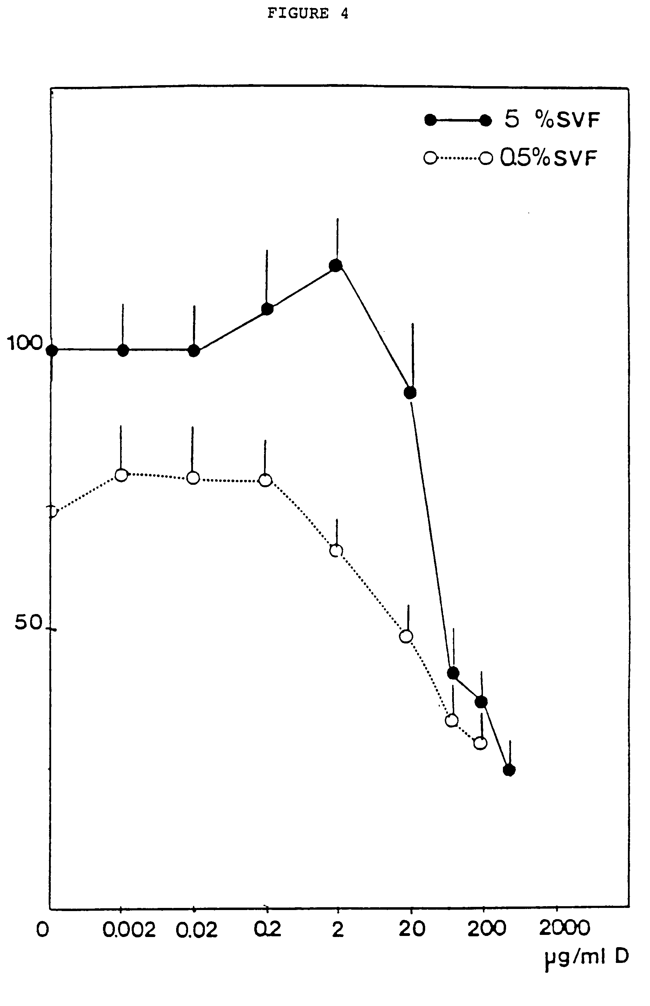

### EXEMPLE 4 (exemple de comparaison) : effets du dextrane substitué D₆CM₇₆B₀S₁₄ (Figure 4)

En présence de 5% (●) de SVF, on observe une stimulation de 15% (P<O,01) de l'incorporation pour une concentration de dextrane substitué égale à 2 µg/ml. Au delà, et pour une concentration de dextrane substitué de 100 à 200 µg/ml, on observe 60% (P<O,001) d'inhibition, le maximum étant de 75% (P<O,001) en présence d'une concentration de dextrane substitué de 400 µg/ml.

En présence de 0,5% de SVF (○), on observe d'abord une activation, puis une légère inhibition de l'ordre de 15% (P<O,05) en présence de 2 µg/ml du dérivé testé. Cette inhibition est de 30% (P<O,001) pour 20 µg/ml et de 53% (P<O,001) pour 100 et 200 µg/ml.

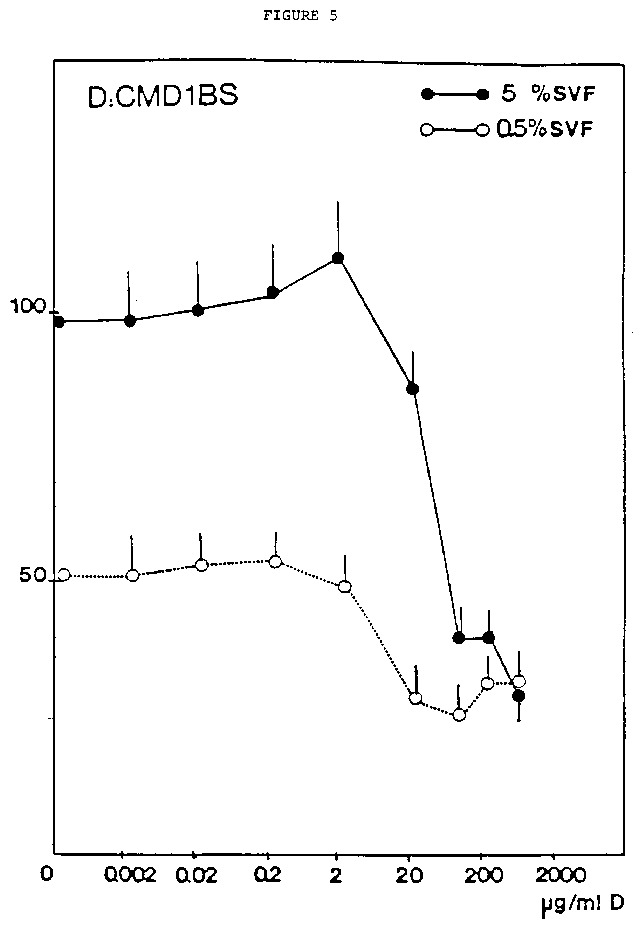

### EXEMPLE 5 : effets du dextrane substitué conforme à l'invention, D₁₁CM₆₀B_{O}S₂₉ (Figure 5)

En présence de 5% de SVF (●), on observe une légère stimulation (10%) (P<O,10) (N.S.) de l'incorporation pour une concentration de 2 µg/ml de dextrane substitué, puis une inhibition. A 100 µg/ml l'inhibition est de 20% (P<O,01) ; à 200 µg/ml, elle est de 60% (P<O,001), et de 75% (P<O,001) pour 400 µg/ml.

En présence de 0,5% (○) de sérum, on n'observe, jusqu'à une concentration de 2 µg/ml aucune modification de la croissance ; en revanche, on observe une inhibition de 50% (P<O,001) en présence de 20 µg/ml du dérivé testé ; cette inhibition reste quasiment constante aux plus fortes concentrations testées.

### CONCLUSION

L'héparine et le dextrane natif T 40 n'induisent presque aucune modification de l'incorporation de la thymidine au niveau des "chondrocytes" tumoraux, quelles que soient les concentrations de ces dérivés utilisées.

En présence d'une concentration de 2 µg/ml de D_{O}CM₈₄B₂₁S_{O} on enregistre une stimulation de 25% de la synthèse d'ADN.

En présence de dérivés substitués de type DCMBS, et en présence de 5% SVF, on observe toujours une légère stimulation de l'incorporation de thymidine pour une concentration de 2 µg/ml. Cette stimulation est de 15% pour le D₆CM₇₆B₀S₁₄, alors qu'elle n'est que de 10%, à la limite du significatif, pour le dérivé D₁₁CM₆₀B₀S₂₉. Pour des concentrations supérieures ou égales à 20 µg/ml c'est une inhibition hautement significative (P<O,001) qui est observée dont le maximum est de 75%, dans le cas du D₁₁CM₆₀B₀S₂₉.

En présence de 0,5% de SVF, les effets du D₁₁CM₆₀B₀S₂₉ et du D₆CM₇₆B₀S₁₄ sont très différents. En effet, à faibles concentrations de D₆CM₇₆B₀S₁₄, on observe une activation de la prolifération cellulaire. Cette activation n'est pas du tout observée, à ces mêmes faibles concentrations, dans le cas de D₁₁CM₆₀B₀S₂₉. En outre, l'inhibition observée pour des concentrations de 20 µg/ml est plus importante dans le cas du D₁₁CM₆₀B₀S₂₉.

### III - ETUDE DES EFFETS DES AGENTS ANTITUMORAUX CONFORMES A L'INVENTION SUR LA CROISSANCE DE LIGNEES CELLULAIRES NON TUMORALES D'ORIGINE HUMAINE (CHONDROCYTES ET FIBROBLASTES DE PEAU).

### EXEMPLE 6 : effets des agents antitumoraux conformes à l'invention sur les chondrocytes normaux

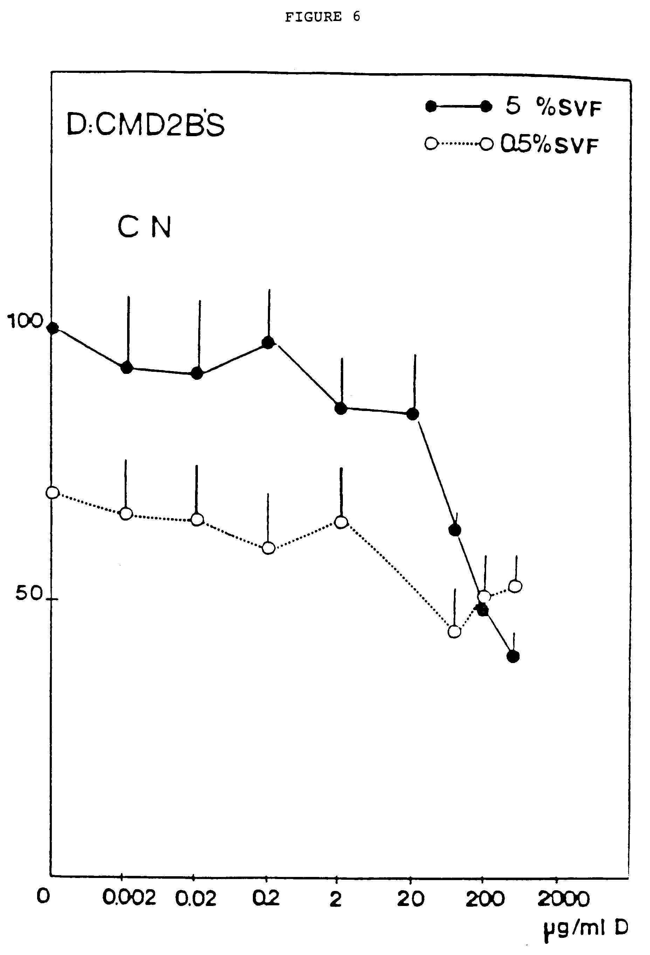

Le dérivé D₁₁CM₆₀B₀S₂, conforme à l'invention, a été testé sur des chondrocytes issus de tête fémorale humaine normale, en parallèle avec le dérivé D₆CM₇₆B₀S₁₄. Les résultats sont identiques pour les deux dérivés étudiés. La Figure 6 représente l'effet du D₆CM₇₆B₀S₁₄ Pour des concentrations de 2 µg/ml à 20 µg/ml, l'incorporation de thymidine tritiée est peu modifiée. Pour des concentrations de 100 µg/ml, l'inhibition de l'incorporation est de 36% (P<O,001) en présence de 5% de SVF et atteint la valeur de 51% (P<O,001) pour les concentrations de 200 et 400 µg/ml. Pour 0,5% de SVF (○), les taux d'inhibition sont de l'ordre de 25% (P<O,01).

### EXEMPLE 7 : effets des agents antitumoraux conformes à l'invention sur les fibroblastes de peau.

Les effets des dérivés D₁₁CM₆₀B₀S₂₉ et D₆CM₇₆B₀S₁₄ ont été étudiés sur les fibroblastes de peau.

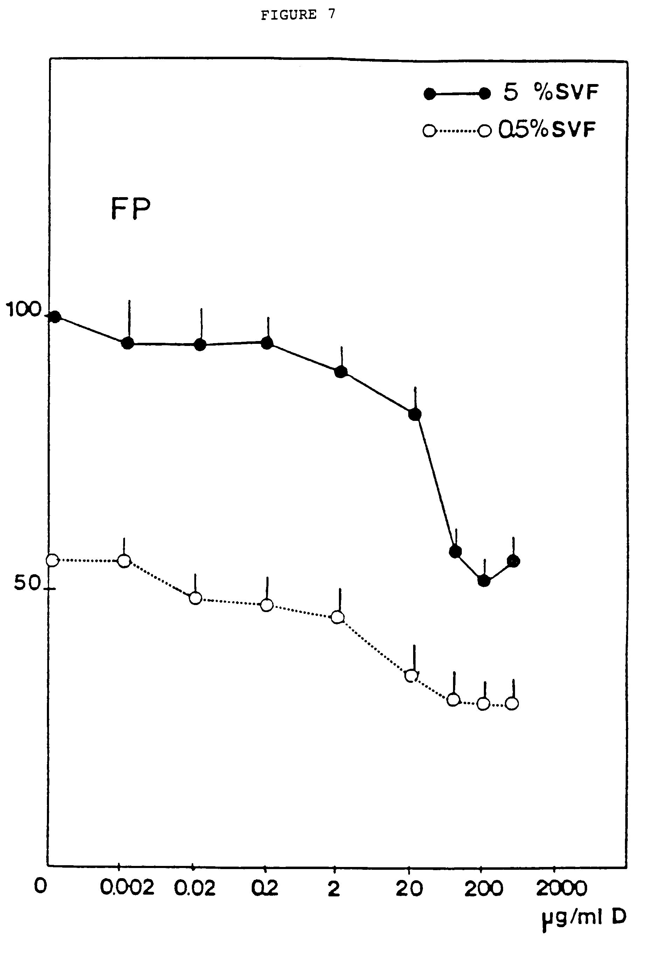

La Figure 7 représente l'effet du D₆CM₇₆B₀S₁₄.

Comme dans le cas des chondrocytes normaux, les effets sont identiques pour les 2 dérivés ; l'incorporation de thymidine tritiée est peu modifiée pour des concentrations de dérivés allant de 2 à 20 µg/ml, et ce, en présence des deux concentrations de sérum testées 5% (●) et 0,5% (○). Pour les fortes concentrations des deux dérivés (100 à 400 µg/ml), le taux d'inhibition est d'environ 50% (P<O,001).

### CONCLUSION

La sensibilité des lignées humaines normales et tumorales à l'action des agents antitumoraux conformes à l'invention est très différente. Le taux d'inhibition pour les lignées normales de chondrocytes et de fibroblastes est au maximum de 50%, et ce, pour des concentrations très élevées de dérivés conformes à l'invention, alors que l'inhibition maximale pour les chondrocytes tumoraux est de 75%, pour des concentrations beaucoup plus faibles.

### IV - MODULATION DE LA PROLIFERATION DES CELLULES TUMORALES EN PRESENCE DES DERIVES CONFORMES A L'INVENTION

### EXEMPLE 8 :

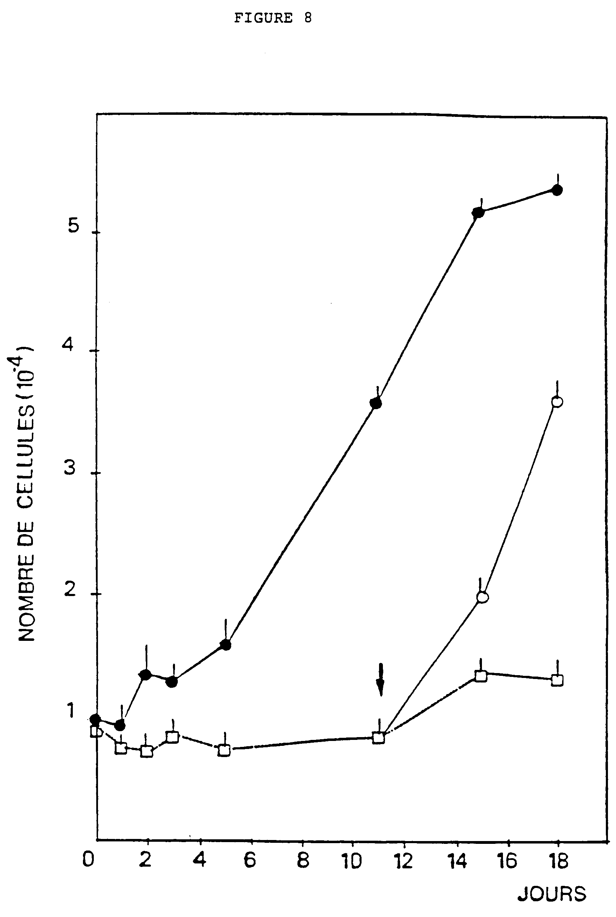

Des cellules de chondrosarcome humain de type II sont ensemencées sur des boîtes de 4 cupules de 2 cm² en présence de 5% SVF (●) d'une part (contrôle), et, en parallèle, en présence de 5% SVF (□) et de D₁₁CM₆₀B₀S₂₉ à une concentration de 200 µg/ml. Le milieu est changé tous les 3 jours. Comme le montre la Figure 8, en présence de D₁₁CM₆₀B₀S₂₉ les cellules restent quiescentes; par contre lorsque le D₁₁CM₆₀B₀S₂₉ est ôté du milieu de culture au bout de 12 jours (○), la croissance cellulaire reprend, ce qui montre que l'effet du D₁₁CM₆₀B₀S₂₉ est réversible.

### CONCLUSION

A une concentration de 200 µg/ml de D₁₁CM₆₀B₀S₂₉, pour laquelle on enregistre seulement 75% d'inhibition de l'incorporation de thymidine, la prolifération cellulaire est totalement inhibée. Cette inhibition par le D₁₁CM₆₀B₀S₂₉ n'est pas cytotoxique car parfaitement réversible.

### EXEMPLE 9 :

Dans le but de mettre en évidence une action inhibitrice des dextranes substitués, les dérivés ont été testés sur le développement d'un chondrosarcome proche du chondrosarcome humain différencié, le chondrosarcome greffable du rat de Laboratoire. Ces expériences ont été faites in-vivo.

Des rats d'environ 3 mois sont greffés avec ce chondrosarcome (un petit fragment de cette tumeur est implanté stérilement sous la peau du flanc de l'animal). Plusieurs séries de 10 animaux ont été utilisées.

La solution de dextrane substitué est alors injectée localement dans la partie périphérique de la tumeur quand celle-ci a atteint un diamètre d'environ 20 mm.

La croissance totale de la tumeur est ralentie par rapport aux témoins en ce sens qu'on observe un développement moins rapide dans la région de la tumeur qui a reçu l'injection de dextrane substitué.

Nous avons alors cherché à visualiser l'effet en ajoutant du charbon micronisé aux solutions de dextrane substitué avant injection dans la partie périphérique du chondrosarcome. Dans ces conditions, on peut facilement repérer la zone de la tumeur qui a reçu l'injection. On observe alors que la zone de la tumeur où la croissance est ralentie correspond à celle qui a reçu l'injection de dextrane substitué.

## Revendications (Revendications pour l'(les) Etat(s) contractant(s) suivant(s): AT, BE, CH, DE, DK, FR, GB, IT, LI, LU, NL, SE)

1. Utilisation d'un dérivé de dextrane constitué par une chaîne polysaccharidique substituée par des groupes carboxyméthyle et carboxyméthylbenzylamide sulfonate, lequel dérivé est désigné par la formule générale D_{X}CM_{Y}BS_{Z} dans laquelle
X représente le nombre moyen d'unités saccharidiques non substituées pour 100 unités saccharidiques,
Y représente le nombre moyen de groupes carboxyméthyle pour 100 unités saccharidiques,
Z représente le nombre moyen de groupes carboxyméthylbenzylamide sulfonate pour 100 unités saccharidiques,
et X est inférieur ou égal à 50, Y est compris entre 10 et 90, et Z est compris entre 15 et 35, pour l'obtention d'un agent inhibiteur de la croissance des cellules tumorales.

2. Dérivé de dextrane, de formule D_{X}CM_{Y}BS_{Z} telle que définie ci-dessus, dans laquelle X est égal à 11, Y est égal à 60 et Z est égal à 29, pour l'utilisation comme agent inhibiteur de la croissance des cellules tumorales.

3. Dérivé de dextrane, de formule D_{X}CM_{Y}BS_{Z} telle que définie définie ci-dessus, dans laquelle X est égal à 47, Y est égal à 17 et Z est égal à 24, pour l'utilisation comme agent inhibiteur de la croissance des cellules tumorales.

4. Médicaments caractérisés en ce qu'ils comprennent en tant que principe actif un dérivé de dextrane tel que défini dans l'une quelconque des Revendications 2 ou 3.

## Revendications (Revendications pour l'(les) Etat(s) contractant(s) suivant(s): ES, GR)

1. Procédé d'obtention d'un agent inhibiteur de la croissance des cellules tumorales caractérisé en ce que l'on met en oeuvre, en tant que constituant essentiel dudit agent, un dérivé de dextrane constitué par une chaîne polysaccharidique substituée par des groupes carboxyméthyle et carboxyméthylbenzylamide sulfonate, lequel dérivé est désigné par la formule générale D_{X}CM_{Y}BS_{Z} dans laquelle
X représente le nombre moyen d'unités saccharidiques non substituées pour 100 unités saccharidiques,
Y représente le nombre moyen de groupes carboxyméthyle pour 100 unités saccharidiques,
Z représente le nombre moyen de groupes carboxyméthylbenzylamide sulfonate pour 100 unités saccharidiques,
et X est inférieur ou égal à 50, Y est compris entre 10 et 90, et Z est compris entre 15 et 35.

2. Procédé selon la revendication 1, caractérisé en ce que le dérivé de dextrane mis en oeuvre est désigné par la formule D_{X}CM_{Y}BS_{Z} telle que définie ci-dessus, dans laquelle X est égal à 11, Y est égal à 60 et Z est égal à 29.

3. Procédé selon la revendication 1, caractérisé en ce que le dérivé de dextrane mis en oeuvre est désigné par la formule D_{X}CM_{Y}BS_{Z} telle que définie ci-dessus, dans laquelle X est égal à 47, Y est égal à 17 et Z est égal à 24.

4. Procédé d'obtention d'un médicament, caractérisé en ce que l'on incorpore, en tant que principe actif audit médicament, un dérivé de dextrane tel que défini dans l'une quelconque des Revendications 2 ou 3.

## Claims (Claims for the following Contracting State(s): AT, BE, CH, DE, DK, FR, GB, IT, LI, LU, NL, SE)

1. Use of a dextrane derivative constituted by a polysaccharide chain substituted by carboxymethyl and carboxymethyl benzyl amide sulphonate groups, the said derivative being designated by the general formula D_{X}CM_{Y}BS_{Z} in which
X represents the average number of non-substituted saccharide units per 100 saccharide units,
Y represents the average number of carboxymethyl groups per 100 saccharide units,
Z represents the average number of carboxymethyl benzyl amide sulphonate groups per 100 saccharide groups
and X is less than or equal to 50, Y is comprised between 10 and 90 and Z is comprised between 15 and 35 in order to obtain an agent inhibiting the growth of tumoral cells.

2. A dextrane derivative to formula D_{X}CM_{Y}BS_{Z} as defined hereinabove in which X is equal to 11, Y is equal to 60 and Z is equal to 29, for use as an agent inhibiting the growth of tumoral cells.

3. A dextrane derivative to formula D_{X}CM_{Y}BS_{Z} as defined hereinabove in which X is equal to 47, Y is equal to 17 and Z is equal to 24, for use as an agent inhibiting the growth of tumoral cells.

4. Medicaments characterised in that they comprise as an active principle a dextrane derivative as defined in any one of Claims 2 or 3.

## Claims (Claims for the following Contracting State(s): ES, GR)

1. A method of obtaining an agent inhibiting the growth of tumoral cells, characterised in that as an essential constituent of the said agent, use is made of a dextrane derivative constituted by a polysaccharide chain substituted by carboxymethyl and carboxymethyl benzyl amide sulphonate groups, the said derivative being designated by the general formula D_{X}CM_{Y}BS_{Z} in which
X represents the average number of non-substituted saccharide units per 100 saccharide units,
Y represents the average number of carboxymethyl groups per 100 saccharide units,
Z represents the average number of carboxymethyl benzyl amide sulphonate groups per 100 saccharide units
and X is less than or equal to 50, Y is comprised between 10 and 90 and Z is comprised between 15 and 35.

2. A method according to Claim 1 characterised in that the dextrane derivative employed is designated by the formula D_{X}CM_{Y}BS_{Z} as defined hereinabove in which X is equal to 11, Y is equal to 60 and Z is equal to 29.

3. A method according to Claim 1 characterised in that the dextrane derivative employed is designated by the formula D_{X}CM_{Y}BS_{Z} as defined hereinabove in which X is equal to 47, Y is equal to 17 and Z is equal to 24.

4. A method of obtaining a medicament characterised in that as an active principle there is incorporated into the said medicament a dextrane derivative as defined in any one of Claims 2 or 3.

## Patentansprüche (Patentansprüche für folgende(n) Vertragsstaat(en): AT, BE, CH, DE, DK, FR, GB, IT, LI, LU, NL, SE)

1. Verwendung eines Dextranderivats, das durch eine Polysaccharidkette mit Carboxymethyl- und Carboxymethylbenzylamid-sulfonatgruppen als Substituenten gebildet wird und der allgemeinen Formel D_{X}CM_{Y}BS_{Z} entspricht, in der
X die mittlere Zahl der nicht substituierten Saccharideinheiten pro 100 Saccharideinheiten,
Y die mittlere Zahl der Carboxymethylgruppen pro 100 Saccharideinheiten und
Z die mittlere Zahl der Carboxymethylbenzylamidsulfonatgruppen pro 100 Saccharideinheiten
bedeuten, wobei X kleiner oder gleich 50 ist, Y zwischen 10 und 90 und Z zwischen 15 und 35 liegt, zur Herstellung eines das Wachstum von Tumorzellen hemmenden Mittels.

2. Dextranderivat der wie vorstehend definierten Formel D_{X}CM_{Y}BS_{Z}, bei der X gleich 11, Y gleich 60 und Z gleich 29 ist, zur Verwendung als Tumorzellenwachstum hemmendes Mittel.

3. Dextranderivat der wie vorstehend definierten Formel D_{X}CM_{Y}BS_{Z}, bei der X gleich 47, Y gleich 17 und Z gleich 24 ist, zur Verwendung als Tumorzellenwachstum hemmendes Mittel.

4. Arzneimittel, dadurch genennzeichnet, daß sie als Wirkstoff ein Dextranderivat der in Anspruch 2 oder 3 definierten Art enthalten.

## Patentansprüche (Patentansprüche für folgende(n) Vertragsstaat(en): ES, GR)

1. Verfahren zur Herstellung eines das Tumorzellenwachstum hemmenden Mittels, dadurch gekennzeichnet, daß man als wesentlichen Bestandteil desselben ein Dextranderivat einsetzt, das durch eine Polysaccharidkette mit Carboxymethyl- und Carboxymethylbenzylamid-sulfonatgruppen als Substituenten gebildet wird und der allgemeinen Formel D_{X}CM_{Y}BS_{Z} entspricht, in der
X die mittlere Zahl der nicht substituierten Saccharideinheiten pro 100 Saccharideinheiten,
Y die mittlere Zahl der Carboxymethylgruppen pro 100 Saccharideinheiten und
Z die mittlere Zahl der Carboxymethylbenzylamidsulfonatgruppen pro 100 Saccharideinheiten
bedeuten, wobei X kleiner oder gleich 50 ist, Y zwischen 10 und 90 und Z zwischen 15 und 35 liegt.

2. Verfahren nach Anspruch 1, dadurch gekennzeichnet, daß das eingesetzte Dextranderivat der wie vorstehend definierten Formel D_{X}CM_{Y}BS_{Z} entspricht, bei der X gleich 11, Y gleich 60 und Z gleich 29 ist.

3. Verfahren nach Anspruch 1, dadurch gekennzeichet, daß das eingesetzte Dextranderivat der wie vorstehend definierten Formel D_{X}CM_{Y}BS_{Z} entspricht, bei der X gleich 47, Y gleich 17 und Z gleich 24 ist.

4. Verfahren zur Herstellung eines Arzneimittels, dadurch gekennzeichnet, daß man in dasselbe als Wirkstoff ein Dextranderivat wie in einem der Ansprüche 2 oder 3 definiert einbringt.
